# EUROPÄISCHE PATENTANMELDUNG

(11) **EP 4 382 057 A1**
(43) Veröffentlichungstag der Anmeldung: **12.06.2024**
(21) Anmeldenummer: 23214572.2
(22) Anmeldetag: 06.12.2023
(51) Int. Cl.: A61B 17/34, A61B 1/00, A61B 17/06, A61B 10/00, A61B 17/00

(54) **VORRICHTUNG ZUM ZUFÜHREN VON OPERATIONSMATERIAL IN EIN ENDOSKOPISCHES OPERATIONSGEBIET BZW. ZUM ENTFERNEN VON OPERATIONSMATERIAL AUS EINEM ENDOSKOPISCHEN OPERATIONSGEBIET**

(30) Priorität: 08.12.2022 DE 102022132716
(71) Anmelder: Karl Storz SE & Co. KG, 78532 Tuttlingen (DE)
(72) Erfinder: Schneider, Janosz, 78532 Tuttlingen (DE); Stefan, Jochen, 78532 Tuttlingen (DE)

(57) **Zusammenfassung**

Die Erfindung betrifft eine Vorrichtung (1) zum Zuführen von Operationsmaterial in ein endoskopisches Operationsgebiet bzw. zum Entfernen von Operationsmaterial aus einem endoskopischen Operationsgebiet, mit einem Trokar (3) sowie einem durch den Trokar (3) in das Operationsgebiet einführbaren medizinischen Instrument. Eine Vorrichtung (1) zum Zuführen von Operationsmaterial in ein endoskopisches Operationsgebiet bzw. zum Entfernen von Operationsmaterial aus einem endoskopischen Operationsgebiet, die bei einfacher Handhabung ein sicheres Zuführen und Entfernen von Operationsmaterial ermöglicht, ist erfindungsgemäß gekennzeichnet durch ein am proximalen Ende (4) des Trokars (3) angeordnetes beladbares Magazin (5) zur Aufnahme von Material-Containern (6), die mittels des medizinischen Instruments (7) durch den Trokar (3) in das Operationsgebiet einführbar bzw. aus diesem entfernbar sind.

## Beschreibung

Die Erfindung betrifft eine Vorrichtung zum Zuführen von Operationsmaterial in ein endoskopisches Operationsgebiet bzw. zum Entfernen von Operationsmaterial aus einem endoskopischen Operationsgebiet, mit einem Trokar sowie einem durch den Trokar in das Operationsgebiet einführbaren medizinischen Instrument.

Während einer endoskopischen Operation ist es immer wieder erforderlich, Operationsmaterial, wie beispielsweise Nahtmaterial, in das Operationsgebiet einzuführen sowie Operationsmaterial, wie beispielsweise Gewebeproben, aus dem Operationsgebiet zu entfernen. In der Praxis erfolgt das Zuführen und Entnehmen von Operationsmaterial mittels eines medizinischen Instruments, beispielsweise einer Zange, das durch einen Trokar in das Operationsgebiet eingeführt bzw. aus diesem entfernt wird.

Diese Vorgehensweise zum Einbringen und Entfernen von Operationsmaterial hat sich in der endoskopischen Chirurgie zwar durchaus bewährt, jedoch bedarf es je nach Operationsmaterial spezieller medizinischer Instrumente, um dieses in das Operationsgebiet einzuführen bzw. aus diesem zu entfernen, und darüber hinaus kann es bei der Verwendung größerer Trokare zu Insufflationsverlusten über diese Trokare kommen. Beim Ausbringen von Gewebeproben aus dem Operationsgebiet kann es zu Kontamination und / oder dem Verlust der Gewebeprobe kommen.

Davon ausgehend liegt der Erfindung die **Aufgabe** zugrunde, eine Vorrichtung zum Zuführen von Operationsmaterial in ein endoskopisches Operationsgebiet bzw. zum Entfernen von Operationsmaterial aus einem endoskopischen Operationsgebiet zu schaffen, die bei einfacher Handhabung ein sicheres Zuführen und Entfernen von Operationsmaterial ermöglicht.

Die **Lösung** dieser Aufgabenstellung ist erfindungsgemäß gekennzeichnet durch ein am proximalen Ende des Trokars angeordnetes beladbares Magazin zur Aufnahme von Material-Containern, die mittels des medizinischen Instruments durch den Trokar in das Operationsgebiet einführbar bzw. aus diesem entfernbar sind.

Mit der erfindungsgemäßen Vorrichtung ist es möglich, unterschiedlichste Operationsmaterialien sicher und einfach mittels nur eines medizinischen Instruments dem Operationsgebiet zuzuführen bzw. aus diesem zu entfernen. Die Material-Container werden außerhalb des Patientenkörpers mit dem entsprechenden Operationsmaterial bestückt und dann mittels eines medizinischen Instruments durch den Trokar in das Operationsgebiet eingeführt. Ebenso können beispielsweise Gewebeproben über einen leer in das Operationsgebiet eingeführten Material-Container mittels des medizinischen Instruments durch den Trokar aus dem Operationsgebiet entfernt werden.

Um die Vorrichtung besonders flexibel auszugestalten, sind die Material-Container in einem am proximalen Ende des Trokars angeordneten Magazin gelagert, so dass leere und auch mit unterschiedlichen Operationsmaterialien bestückte Material-Container jederzeit einsatzbereit zur Verfügung stehen und mittels des medizinischen Instruments dem Operationsgebiet zugeführt werden können. Mit der erfindungsgemäßen Vorrichtung ist es ohne das medizinische Instrument zu wechseln jederzeit möglich, einen Material-Container in das Magazin zurückzubringen und einen anderen Material-Container in das Operationsgebiet einzubringen.

Gemäß einer bevorzugten Ausführungsform der Erfindung wird vorgeschlagen, dass das Magazin als um die Längsachse des Trokars drehbare Trommel ausgebildet ist. Die Verwendung eines derartigen Revolvermagazins ist besonders platzsparend und leicht zu handhaben.

Mit einer alternativen Ausführungsform der Erfindung wird vorgeschlagen, dass das Magazin als quer zur Längsachse des Trokars verschiebbarer Schlitten ausgebildet ist.

Das Magazin zur Aufnahme der Material-Container ist erfindungsgemäß vorteilhafterweise motorisch antreibbar, wodurch ein schnelles und lagegerechtes Zuordnen des erforderlichen Material-Containers gewährleistet wird.

Um den jeweiligen Material-Container mittels des medizinischen Instruments durch den Trokar in das Operationsgebiet einführen zu können, wird gemäß einer praktischen Ausführungsform der Erfindung vorgeschlagen, dass jeder Material-Container mittels eines Rastmechanismus an einer distalen Instrumentenspitze des medizinischen Instruments festlegbar ist. Die Ausbildung des Kopplungssystems als Rastmechanismus stellt eine fertigungstechnisch einfache und darüber hinaus sicher zu handhabende Verbindung dar.

Zur Ausbildung des Rastmechanismus wird mit der Erfindung vorgeschlagen, dass der Rastmechanismus aus einem an der distalen Instrumentenspitze des medizinischen Instruments angeordneten Rastelement und einer korrespondierenden Rastaufnahme am Material-Container besteht. Über die beiden miteinander korrespondierenden Bauteile Rastelement und Rastaufnahme erfolgt eine sichere Kopplung zwischen der distalen Instrumentenspitze des medizinischen Instruments und dem in das Operationsgebiet einzuführenden Material-Container.

Weiterhin wird mit der Erfindung vorgeschlagen, dass das an der distalen Instrumentenspitze des medizinischen Instruments angeordnete Rastelement um die Längsachse des medizinischen Instruments drehbar ist. Durch die Verdrehbarkeit des Rastelements kann eine schnelle und sichere Kopplung mit der Rastaufnahme des Material-Containers erleichtert werden.

Mit einer alternativen erfindungsgemäßen Ausführungsform zur Ausbildung des Kopplungssystem zwischen dem Material-Container einerseits und der distalen Instrumentenspitze des medizinischen Instruments andererseits wird vorgeschlagen, dass jeder Material-Container mittels eines magnetischen Kopplungssystems an der distalen Instrumentenspitze des medizinischen Instruments festlegbar ist.

Um das Operationsmaterial sicher und kontaminationsfrei in das Operationsgebiet einführen zu können bzw. aus diesem entfernen zu können, wird mit der Erfindung vorgeschlagen, dass jeder Material-Container eine verschließbare Öffnung zum Befüllen und / oder Entleeren des Material-Containers aufweist. Das Öffnen und Schließen der Öffnung des Material-Containers erfolgt erfindungsgemäß vorteilhafterweise über das mit dem Material-Container koppelbare medizinische Instrument, so dass es keines weiteren medizinischen Instruments bedarf, um den in das Operationsgebiet eingeführten Material-Container zu öffnen.

Gemäß einer bevorzugten Ausführungsform der Erfindung wird vorgeschlagen, dass die mit dem Material-Container koppelbare distale Instrumentenspitze des medizinischen Instruments über mindestens ein Gelenk gegenüber der Längsachse des medizinischen Instruments abwinkelbar ist. Durch die Abwinkelbarkeit der mit dem Material-Container bestückbaren distalen Instrumentenspitze des medizinischen Instruments kann das in dem Material-Container befindliche Operationsmaterial auch in kleinen Cavitäten angereicht und entnommen werden.

Alternativ oder zusätzlich zur Abwinklung der mit dem Material-Container koppelbaren distalen Instrumentenspitze des medizinischen Instruments wird mit der Erfindung vorgeschlagen, dass die mit dem Material-Container koppelbare distale Instrumentenspitze des medizinischen Instruments um die Längsachse des medizinischen Instruments rotierbar ausgebildet ist. Hierdurch kann die Möglichkeit der lagegenauen Zuführung des Operationsmaterials noch weiter verbessert werden.

Gemäß einer praktischen Ausführungsform der Erfindung wird vorgeschlagen, dass das die erfindungsgemäße Vorrichtung mit einem medizinischen Telemanipulationsroboter koppelbar ist.

Alternativ wird mit der Erfindung vorgeschlagen, dass das die Vorrichtung mit manuell bedienbaren medizinischen Instrumenten koppelbar ist

Weitere Merkmale und Vorteile der Erfindung ergeben sich anhand der zugehörigen Zeichnungen, in denen zwei Ausführungsbeispiele einer erfindungsgemäßen Vorrichtung zum Zuführen von Operationsmaterial in ein endoskopisches Operationsgebiet nur beispielhaft dargestellt sind, ohne die Erfindung auf diese Ausführungsbeispiele zu beschränken. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

In den Zeichnungen zeigt:
- Fig. 1: eine perspektivische Draufsicht auf eine erste Ausführungsform einer erfindungsgemäßen Vorrichtung zum Zuführen von Operationsmaterial in ein endoskopisches Operationsgebiet bzw. zum Entfernen von Operationsmaterial aus einem endoskopischen Operationsgebiet,
- Fig. 2: eine Seitenansicht der Darstellung gemäß Fig. 1,
- Fig. 3: einen teilweisen Längsschnitt durch Darstellung gemäß Fig. 2,
- Fig. 4: einen vergrößerte Ansicht des Details IV gemäß Fig. 1,
- Fig. 5: eine Rückansicht der Darstellung gemäß Fig. 4,
- Fig. 6: zwei unterschiedliche perspektivische Ansichten des medizinischen Instruments sowie eines Material-Containers,
- Fig. 7: einen Längsschnitt durch das medizinische Instrument sowie einen Material-Container gemäß Fig. 6 und
- Fig. 8: eine perspektivische Draufsicht gemäß Fig. 1, jedoch eine zweite Ausführungsform einer erfindungsgemäßen Vorrichtung darstellend.

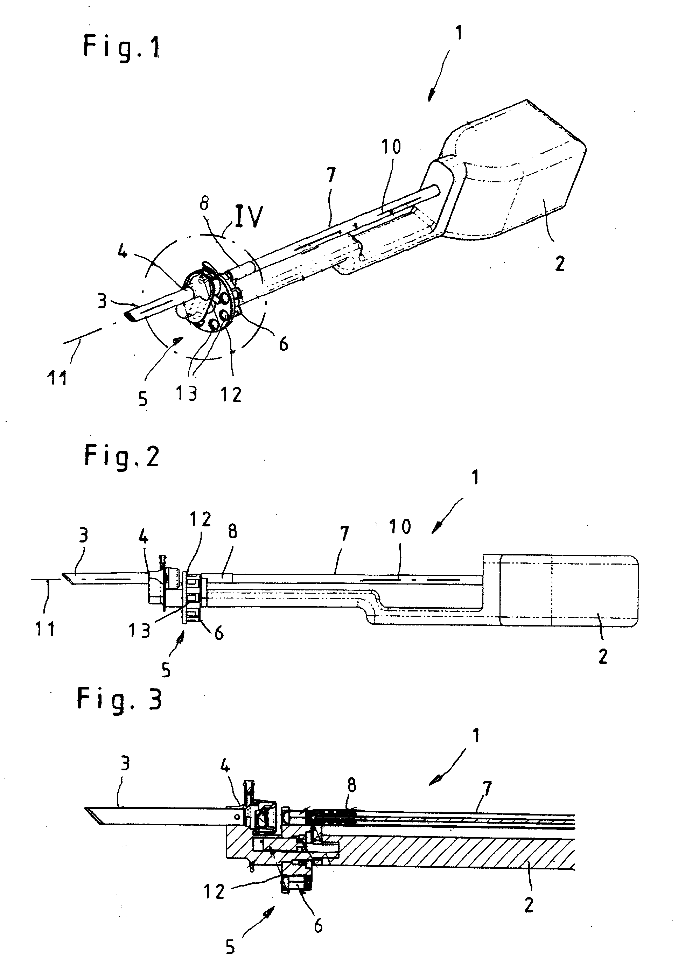

Die Abbildung Fig. 1 zeigt eine Vorrichtung 1 zum Zuführen von Operationsmaterial in ein endoskopisches Operationsgebiet bzw. zum Entfernen von Operationsmaterial aus einem endoskopischen Operationsgebiet. Bei der in Fig. 1 dargestellten Ausführungsform ist die Vorrichtung 1 mit einem medizinischen Telemanipulationsroboter 2 gekoppelt.

Alternativ ist es selbstverständlich auch möglich, die nachfolgend genauer beschriebene Vorrichtung 1 mit weiteren manuell bedienbaren medizinischen Instrumenten zu koppeln.

Die Vorrichtung 1 besteht im Wesentlichen aus einem Trokar 3, einem am proximalen Ende 4 des Trokars 3 angeordneten beladbaren Magazin 5 zur Aufnahme von Material-Containern 6 sowie einem medizinischen Instrument 7, mit Hilfe dessen Material-Container 6 durch den Trokar 3 in das Operationsgebiet einführbar bzw. aus diesem entfernbar sind.

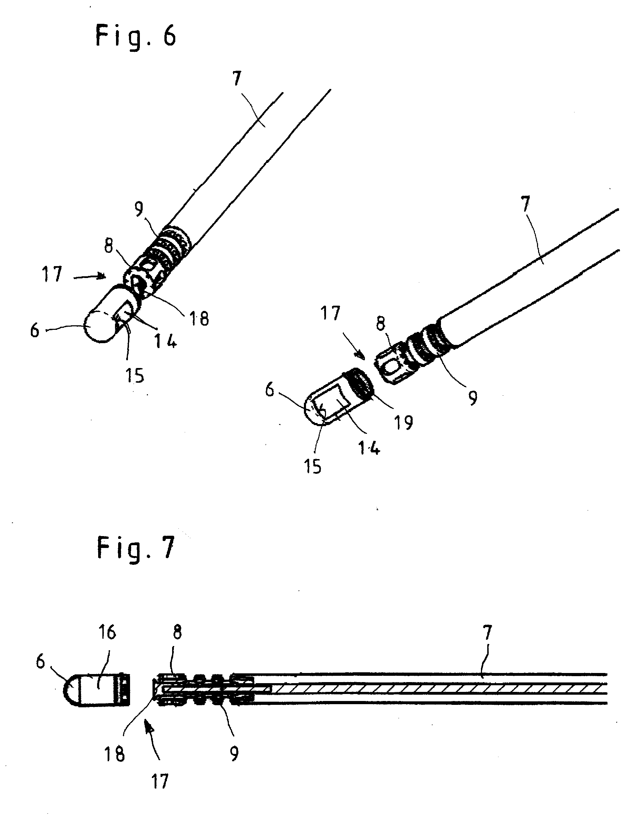

Wie insbesondere aus den Abbildungen Fig. 6 und 7 ersichtlich, weist das medizinische Instrument 7 eine distale Instrumentenspitze 8 auf, die über mindestens ein Gelenk 9 gegenüber der Längsachse 10 des medizinischen Instruments 7 abwinkelbar ist.

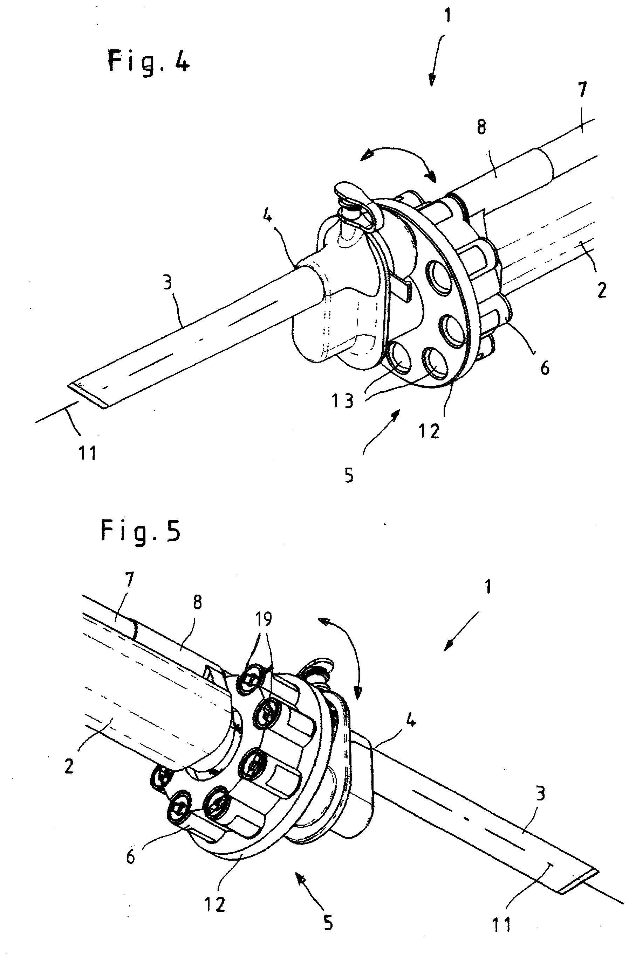

Bei der in den Abbildungen Fig. 1 bis Fig. 5 dargestellten ersten Ausführungsform der Vorrichtung 1 ist das am proximalen Ende 4 des Trokars 3 angeordnete Magazin 5 zur Aufnahme der Material-Container 6 als um die Längsachse 11 des Trokars 3 drehbare Trommel 12 ausgebildet, wie dies den Pfeilen in den Abbildungen Fig. 4 und Fig. 5 zu entnehmen ist.

In dem als Trommel 12 ausgebildeten Magazin 5 sind mehrere Aufnahmefächer 13 zur Aufnahme jeweils eines Material-Containers 6 ausgebildet. Die Material-Container 6 selber sind als hohle zylindrische Kapseln ausgebildet, in deren Mantelflächen eine über eine Klappe 14 verschließbare Öffnung 15 ausgebildet ist, über die der Containerraum 16 eines jeden Material-Containers 6 befüllbar und wieder entleerbar ist. Das Öffnen und Schließen der Öffnung 15 mittels der Klappe 14 erfolgt vorteilhafterweise über das medizinische Instrument 7.

Die in die Aufnahmefächer 13 des Magazins 5 einsetzbaren Material-Container 6 können je nach Einsatz- bzw. Verwendungszweck bereits mit notwendigem Operationsmaterial, wie beispielsweise mit Nahtmaterial vorkonfektionierte Nadeln, bestückt sein oder aber auch leer sein, wenn beispielsweise Gewebeproben aus dem Operationsgebiet zu weiteren Untersuchungen aus dem Körper des Patienten nach außen ausgeführt werden sollen. Die Verwendung der Material-Container 6 hat den Vorteil, dass ab Werk bestückte Material-Container 6 ohne Kontamination in das Magazin 5 geladen werden können. Auch bei der Gewebeentnahme ist die Verwendung des Material-Containers 6 vorteilhaft, da es nicht mehr zu einer Kontamination und / oder dem Verlust der Gewebeprobe kommen kann.

Zum Einführen eines Material-Containers 6 durch den Trokar 3 in das Operationsgebiet sind das distale Ende der Instrumentenspitze 8 einerseits und das proximale Ende eines Material-Containers 6 miteinander koppelbar.

Bei der in den Abbildungen Fig. 5 bis Fig. 7 dargestellten Ausführungsform erfolgt das Koppeln von Material-Container 6 und Instrumentenspitze 8 über einen Rastmechanismus 17, der aus einem an der distalen Instrumentenspitze 8 des medizinischen Instruments 7 angeordneten Rastelement 18 und einer korrespondierenden Rastaufnahme 19 am Material-Container 6 besteht. Wie insbesondere aus der Abbildung Fig. 7 ersichtlich, ist das Rastelement 18 T-förmig ausgebildet. Die in Fig. 5 dargestellten Rastaufnahmen 19 der Material-Container 6 sind als spaltförmige Öffnungen ausgebildet, in die das T-förmige Rastelement 18 einsetzbar ist.

Vorteilhafterweise ist das an der distalen Instrumentenspitze 8 des medizinischen Instruments 7 angeordnete Rastelement 18 um die Längsachse 10 des medizinischen Instruments 7 drehbar, um eine schnelle und sichere Kopplung mit der Rastaufnahme 19 des Material-Containers 6 zu erleichtern. Darüber hinaus kann die Drehbarkeit des Rastelements 18 dazu verwende werden, die Klappe 14 des Material-Containers 6 zwischen einer die Öffnung 15 im Material-Container 6 öffnenden und einer die Öffnung 15 verschließenden Position zu verstellen.

Alternativ zu der dargestellten Kopplung zwischen den Material-Containern 6 einerseits und der distalen Instrumentenspitze 8 des medizinischen Instruments 7 andererseits mittels des Rastmechanismus 17, ist es auch möglich, beispielsweise ein magnetisches Kopplungssystem zur Verbindung der beiden Bauteile vorzusehen.

Nachfolgend wird anhand der Abbildungen Fig. 1 bis Fig. 7 die Verwendung der Vorrichtung 1 beschrieben.

Die Abbildungen Fig. 1 und 2 zeigen die Vorrichtung 1 in einer Ausgangsstellung, in der das medizinische Instrument 7 proximalseitig in Verlängerung des Trokars 3 außerhalb des Trokars 3 angeordnet ist und die Aufnahmefächer 13 des als um die Längsachse 11 des Trokars 3 drehbare Trommel 12 ausgebildeten Magazins 5 mit befüllten und / oder leeren Material-Containern 6 bestückt sind.

Durch Rotation der Trommel 12 um die Längsachse 11 des Trokars 3 wird der zum Einführen in das Operationsgebiet benötigte Material-Container 6 in die Koppelstellung gebracht, in der der benötigte Material-Container 6 in Verlängerung der Längsachse 11 des Trokars 6 vor der distalen Instrumentenspitze 8 des medizinischen Instruments 7 angeordnet ist.

Anschließend wird das medizinische Instrument 7 in Richtung der Längsachse 10 des medizinischen Instruments 7 in distale Richtung hin zum Trokar 3 verschoben, bis das Rastelement 18 der Instrumentenspitze 8 in die Rastaufnehme 19 des Material-Containers 6 eingreift und so das medizinische Instrument 7 und der Material-Container 6 über den Rastmechanismus 17 miteinander gekoppelt sind.

Zum Einführen des Material-Containers 6 in das Operationsgebiet wird nun das medizinische Instrument 7 mit der distalseitig mit dem Material-Container 6 bestückte Instrumentenspitze 8 voran durch den Trokar 6 in das Operationsgebiet im Körper des Patienten eingeführt.

Durch die Abwinkelbarkeit der mit dem Material-Container 6 bestückten distalen Instrumentenspitze 8 des medizinischen Instruments 7 gegenüber der Längsachse 10 des medizinischen Instruments 7 mittels des Gelenks 9 kann das in dem Material-Container 6 befindliche Operationsmaterial auch in kleinen Cavitäten angereicht und entnommen werden. Vorteilhafterweise ist die mit dem Material-Container 6 bestückte distale Instrumentenspitze 8 des medizinischen Instruments 7 zusätzlich um die Längsachse 10 des medizinischen Instruments 7 drehbar, um die Möglichkeit der lagegenauen Zuführung des Operationsmaterials noch weiter zu verbessern.

Am Verwendungspunkt im Operationsgebiet angekommen, wird die die Öffnung 15 des Material-Containers 6 verschließende Klappe 14, beispielsweise durch Drehen des Rastelements 18 um die Längsachse 10 des medizinischen Instruments 7 geöffnet, so dass das im Containerraum 16 gelagerte Operationsmaterial mittels eines nicht dargestellten, über einen anderen Zugang in das Operationsgebiet eingeführten weiteren medizinischen Instruments entnommen werden kann bzw. im Falle eines leeren Material-Containers 6 der Containerraum 16 beispielsweise mit einer Gewebeprobe befüllt werden kann.

Nach erfolgtem Entleeren oder Befüllen des Containerraums 16 des Material-Containers 6 im Operationsgebiet, wird die Öffnung 15 des Material-Containers 6 wieder über die Klappe 14 verschlossen und der Material-Container 6 durch Zurückziehen des medizinischen Instruments 7 durch den Trokar 6 nach proximal wieder aus dem Operationsgebiet und dem Körper des Patienten entfernt, bis der Material-Container 6 wieder im Aufnahmefach 13 des Magazins 5 gelagert ist.

Nachfolgend wird der Rastmechanismus 17, über den der Material-Container 6 und die Instrumentenspitze 8 des medizinischen Instruments 7 miteinander gekoppelt sind, gelöst und das medizinische Instrument 7 in Richtung seiner Längsachse 10 weiter nach proximal gezogen, bis das distalseitige Rastelement 18 der Instrumentenspitze 8 außer Eingriff mit der Rastaufnahme 19 des Material-Containers 6 tritt.

Soll nun ein anderer Material-Container 6 in das Operationsgebiet eingeführt werden, wird die Trommel 12 des Magazins 5 erneut um die Längsachse 11 des Trokars 6 gedreht, bis der neue, zum Einführen in das Operationsgebiet benötigte Material-Container 6 sich in der Koppelstellung befindet, in der dieser Material-Container 6 in Verlängerung der Längsachse 11 des Trokars 6 vor der distalen Instrumentenspitze 8 des medizinischen Instruments 7 angeordnet ist.

Das Drehen des Magazins 5 erfolgt insbesondere bei der Kopplung der Vorrichtung 1 mit einem medizinischen Telemanipulationsroboter 2 motorisch, wodurch eine schnelle und präzise Bereitstellung des jeweils benötigten Material-Containers 6 gewährleistet wird.

Durch die Anordnung der Material-Container 6 in einem mit mehreren Material-Containern 6 bestückbaren Magazin 5 ist es auf einfache Art und Weise möglich, Operationsmaterial sicher und schnell in ein Operationsgebiet einführen bzw. aus diesem wieder entfernen zu können.

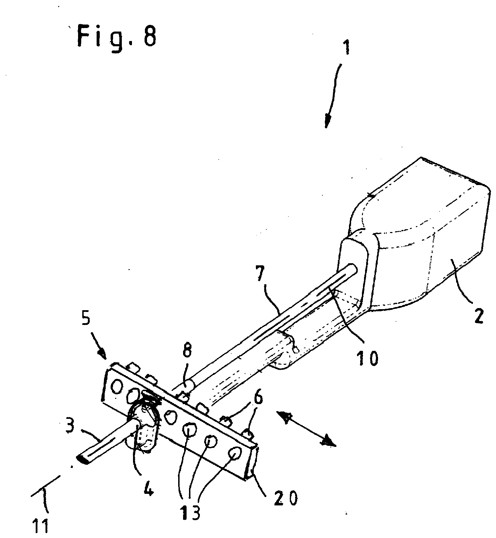

Die Abbildung Fig. 8 zeigt eine alternative Ausführungsform zur Ausbildung des zur Aufnahme der Material-Container 6 dienenden Magazins 5. Wie aus der Abbildung ersichtlich, ist das Magazin 5 bei dieser Ausführungsform als in Richtung des Pfeils quer zur Längsachse 11 des Trokars 3 verschiebbarer Schlitten 20 ausgebildet.

Die Funktionsweise der Vorrichtung 1 mit dem als Schlitten 20 ausgebildeten Magazin 5 entspricht ansonsten der zuvor anhand der mit der drehbaren Trommel 12 ausgestatteten Vorrichtung 1 beschriebenen Vorgehensweise.

In den Zeichnungen ist ein Ausführungsbeispiel der Erfindung dargestellt. Die Zeichnungen, die Beschreibung und die Ansprüche enthalten zahlreiche Merkmale in Kombination. Es versteht sich, dass die genannten Merkmale nicht nur in der jeweils angegebenen Kombination, sondern auch in anderen Kombinationen oder in Alleinstellung verwendbar sind, ohne den Rahmen der vorliegenden Erfindung zu verlassen. Die Erfindung betrifft eine Vorrichtung 1 zum Zuführen von Operationsmaterial in ein endoskopisches Operationsgebiet bzw. zum Entfernen von Operationsmaterial aus einem endoskopischen Operationsgebiet, mit einem Trokar 3 sowie einem durch den Trokar 3 in das Operationsgebiet einführbaren medizinischen Instrument. Eine Vorrichtung 1 zum Zuführen von Operationsmaterial in ein endoskopisches Operationsgebiet bzw. zum Entfernen von Operationsmaterial aus einem endoskopischen Operationsgebiet, die bei einfacher Handhabung ein sicheres Zuführen und Entfernen von Operationsmaterial ermöglicht, ist erfindungsgemäß gekennzeichnet durch ein am proximalen Ende 4 des Trokars 3 angeordnetes beladbares Magazin 5 zur Aufnahme von Material-Containern 6, die mittels des medizinischen Instruments 7 durch den Trokar 3 in das Operationsgebiet einführbar bzw. aus diesem entfernbar sind.

### Bezugszeichenliste

- 1: Vorrichtung
- 2: medizinischer Telemanipulationsroboter
- 3: Trokar
- 4: proximales Ende (Trokar)
- 5: Magazin
- 6: Material-Container
- 7: medizinisches Instrument
- 8: Instrumentenspitze
- 9: Gelenk
- 10: Längsachse (medizinisches Instrument)
- 11: Längsachse (Trokar)
- 12: Trommel
- 13: Aufnahmefach
- 14: Klappe
- 15: Öffnung
- 16: Containerraum
- 17: Rastmechanismus
- 18: Rastelement
- 19: Rastaufnahme
- 20: Schlitten

## Patentansprüche

1. Vorrichtung (1) zum Zuführen von Operationsmaterial in ein endoskopisches Operationsgebiet bzw. zum Entfernen von Operationsmaterial aus einem endoskopischen Operationsgebiet, mit einem Trokar (3) sowie einem durch den Trokar (3) in das Operationsgebiet einführbaren medizinischen Instrument (7),
**gekennzeichnet durch**
ein am proximalen Ende (4) des Trokars (3) angeordnetes beladbares Magazin (5) zur Aufnahme von Material-Containern (6), die mittels des medizinischen Instruments (7) durch den Trokar (3) in das Operationsgebiet einführbar bzw. aus diesem entfernbar sind.

2. Vorrichtung (1) nach Anspruch 1, **dadurch gekennzeichnet, dass** das Magazin (5) als um die Längsachse (11) des Trokars (3) drehbare Trommel (12) ausgebildet ist.

3. Vorrichtung (1) nach Anspruch 1, **dadurch gekennzeichnet, dass** das Magazin (5) als quer zur Längsachse (11) des Trokars (3) verschiebbarer Schlitten (20) ausgebildet ist.

4. Vorrichtung (1) nach einem der Ansprüche 1 bis 3, **dadurch gekennzeichnet, dass** das Magazin (5) motorisch antreibbar ist.

5. Vorrichtung (1) nach einem der Ansprüche 1 bis 4, **dadurch gekennzeichnet, dass** jeder Material-Container (6) mittels eines Rastmechanismus (17) an einer distalen Instrumentenspitze (8) des medizinischen Instruments (7) festlegbar ist.

6. Vorrichtung (1) nach Anspruch 5, **dadurch gekennzeichnet, dass** der Rastmechanismus (17) aus einem an der distalen Instrumentenspitze (8) des medizinischen Instruments (7) angeordneten Rastelement (18) und einer korrespondierenden Rastaufnahme (19) am Material-Container (6) besteht.

7. Vorrichtung (1) nach Anspruch 6, **dadurch gekennzeichnet, dass** das an der distalen Instrumentenspitze (8) des medizinischen Instruments (7) angeordnete Rastelement (18) um die Längsachse (10) des medizinischen Instruments (7) drehbar ist.

8. Vorrichtung (1) nach einem der Ansprüche 1 bis 4, **dadurch gekennzeichnet, dass** jeder Material-Container (6) mittels eines magnetischen Kopplungssystems an der distalen Instrumentenspitze (8) des medizinischen Instruments (7) festlegbar ist.

9. Vorrichtung (1) nach einem der Ansprüche 1 bis 8, **dadurch gekennzeichnet, dass** jeder Material-Container (6) eine verschließbare Öffnung (15) zum Befüllen und / oder Entleeren des Material-Containers (6) aufweist.

10. Vorrichtung (1) nach Anspruch 9, **dadurch gekennzeichnet, dass** das Öffnen und Schließen der Öffnung (15) des Material-Containers (6) über das mit dem Material-Container (6) koppelbare medizinische Instrument (7) erfolgt.

11. Vorrichtung (1) nach einem der Ansprüche 1 bis 10, **dadurch gekennzeichnet, dass** die mit dem Material-Container (6) koppelbare distale Instrumentenspitze (8) des medizinischen Instruments (7) über mindestens ein Gelenk (9) gegenüber der Längsachse (10) des medizinischen Instruments (7) abwinkelbar ist.

12. Vorrichtung (1) nach einem der Ansprüche 1 bis 11, **dadurch gekennzeichnet, dass** die mit dem Material-Container (6) koppelbare distale Instrumentenspitze (8) des medizinischen Instruments (7) um die Längsachse (10) des medizinischen Instruments (7) rotierbar ausgebildet ist.

13. Vorrichtung (1) nach einem der Ansprüche 1 bis 12, **dadurch gekennzeichnet, dass** das die Vorrichtung (1) mit einem medizinischen Telemanipulationsroboter (2) koppelbar ist.

14. Vorrichtung (1) nach einem der Ansprüche 1 bis 12, **dadurch gekennzeichnet, dass** das die Vorrichtung (1) mit manuell bedienbaren medizinischen Instrumenten koppelbar ist.
